# EUROPEAN PATENT APPLICATION

(11) **EP 4 671 353 A1**
(43) Date of publication of application: **31.12.2025**
(21) Application number: 24763948.7
(22) Date of filing: 28.02.2024
(51) Int. Cl.: C12M 1/34, C12Q 1/02, G01N 27/22

(54) **DISTRIBUTION MEASUREMENT DEVICE**

(30) Priority: 28.02.2023 JP 2023029014; 28.02.2023 JP 2023029016
(71) Applicant: Terumo Kabushiki Kaisha, Tokyo, 1510072 (JP)
(72) Inventor: OKUBO, Itaru, Ashigarakami-gun, Kanagawa 259-0151 (JP); LIU, Liming, Ashigarakami-gun, Kanagawa 259-0151 (JP)
(74) Representative: TBK
(86) International application number: PCT/JP2024/007194
(87) International publication number: WO 2024/181469

(57) **Abstract**

A distribution measurement device (10) includes: a plurality of electrodes (36, 136) provided in a bioreactor (12); a selection unit (50, 150) that sequentially selects a pair of the electrodes from the plurality of electrodes; and a measurement unit (52, 152) that sequentially measures a physical quantity between the pair of electrodes selected by the selection unit.

## Description

### Technical Field

The present invention relates to a distribution measurement device for measuring a distribution of cells and the like inside a bioreactor.

### Background Art

JP6381083B discloses a technique for measuring a cell density in a culture medium during cell culture.

### Summary of Invention

To optimize cell culture, a technique for grasping a diffusion state of cells inside a bioreactor is desired.

An object of the invention is to solve the above-described problems.

(1) A distribution measurement device according to the invention includes: a plurality of electrodes provided in a bioreactor; a selection unit that sequentially selects a pair of the electrodes from the plurality of electrodes; and a measurement unit that sequentially measures a physical quantity between the pair of electrodes selected by the selection unit.
(2) In the distribution measurement device according to the item (1), the plurality of electrodes may be disposed along a longitudinal direction of the bioreactor.
   In the invention according to the item (2), a plurality of pairs of electrodes form a plurality of cell concentration measurement ranges along the longitudinal direction in the bioreactor. Therefore, according to the invention, the distribution of cells in the longitudinal direction of the bioreactor can be measured. The distribution of cells in the longitudinal direction of the bioreactor contributes to optimization of cell culture.
(3) In the distribution measurement device according to the item (2), an electrode group including the plurality of electrodes may include a plurality of assembled electrodes, and the plurality of assembled electrodes may be disposed apart from each other along the longitudinal direction of the bioreactor.
(4) In the distribution measurement device according to the item (2) or (3), a longitudinal direction of each of the electrodes may intersect the longitudinal direction of the bioreactor.
(5) In the distribution measurement device according to any one of the items (2) to (4), the physical quantity may be electrostatic capacitance.
(6) In the distribution measurement device according to any one of the items (2) to (5), a hollow fiber may be provided in the bioreactor, and a longitudinal direction of the hollow fiber may be along the longitudinal direction of the bioreactor.
(7) In the distribution measurement device according to any one of the items (2) to (6), the bioreactor may be provided with a plurality of supply and discharge ports, a first supply and discharge port of the plurality of supply and discharge ports may be provided at one end of the bioreactor, and a second supply and discharge port of the plurality of supply and discharge ports may be provided at the other end of the bioreactor.
(8) The distribution measurement device according to any one of the items (2) to (6) may further include: a display control unit that displays, on a display unit, information indicating a distribution of cells in the longitudinal direction of the bioreactor based on the physical quantity measured by the measurement unit.
(9) The distribution measurement device according to any one of the items (2) to (8) may further include: a storage control unit that stores, in a storage unit, information indicating a distribution of cells in the longitudinal direction of the bioreactor based on the physical quantity measured by the measurement unit.
(10) The distribution measurement device according to any one of the items (2) to (9) may further include: a control unit that controls at least one of an inflow rate of a culture medium into the bioreactor and an outflow rate of the culture medium from the bioreactor such that a distribution of cells in the longitudinal direction of the bioreactor becomes uniform.
   In the above configuration, the control unit executes controls such that the distribution of the cells in the longitudinal direction of the bioreactor becomes uniform, based on the distribution of the cells. When the distribution of the cells in the longitudinal direction of the bioreactor is uniform, nutrition is uniformly distributed to the cells inside the bioreactor. Therefore, the above configuration can contribute to the optimization of cell culture.
(11) In the distribution measurement device according to the item (1), a longitudinal direction of the electrode may be along a longitudinal direction of the bioreactor.
   In the invention according to the item (11), the longitudinal direction of the electrode for measuring the physical quantity is along the longitudinal direction of the bioreactor. According to the invention, by sequentially switching a combination of the electrodes, the distribution of cells in the direction intersecting the longitudinal direction of the bioreactor can be measured. The distribution of cells in the direction intersecting the longitudinal direction of the bioreactor contributes to the optimization of cell culture.
(12) In the distribution measurement device according to the item (11), the physical quantity may be electrostatic capacitance.
(13) In the distribution measurement device according to the item (11) or (12), a hollow fiber may be provided in the bioreactor, and a longitudinal direction of the hollow fiber may be along the longitudinal direction of the bioreactor.
(14) The distribution measurement device according to any one of the items (11) to (13) may further include: a display control unit that displays, on a display unit, information indicating a distribution of cells in a direction intersecting the longitudinal direction of the bioreactor based on the physical quantity sequentially measured by the measurement unit.
(15) The distribution measurement device according to any one of the items (11) to (14) may further include: a storage control unit that stores, in a storage unit, information indicating a distribution of cells in a direction intersecting the longitudinal direction of the bioreactor based on the physical quantity sequentially measured by the measurement unit.

According to the invention, the distribution of cells in the longitudinal direction of the bioreactor or the distribution of cells in the direction intersecting the longitudinal direction of the bioreactor can be measured.

### Brief Description of Drawings

[FIG. 1] FIG. 1 is a block diagram of a distribution measurement device according to a first embodiment.
[FIG. 2] FIGS. 2A and 2B are schematic diagrams illustrating an arrangement of a plurality of electrodes in a first arrangement example.
[FIG. 3] FIGS. 3A and 3B are schematic diagrams illustrating an arrangement of a plurality of electrodes in a second arrangement example.
[FIG. 4] FIGS. 4A and 4B are schematic diagrams illustrating arrangements of one assembled electrode in a third arrangement example.
[FIG. 5] FIG. 5 is a schematic diagram illustrating an arrangement of one assembled electrode in a fourth arrangement example.
[FIG. 6] FIGS. 6A and 6B are schematic diagrams illustrating arrangements of a plurality of electrodes in a fifth arrangement example.
[FIG. 7] FIGS. 7A and 7B are schematic diagrams illustrating arrangements of a plurality of electrodes in the fifth arrangement example.
[FIG. 8] FIGS. 8A and 8B are schematic diagrams illustrating arrangements of a plurality of electrodes in a sixth arrangement example.
[FIG. 9] FIG. 9 is a schematic diagram illustrating an arrangement of a plurality of electrodes in a seventh arrangement example.
[FIG. 10] FIGS. 10A and 10B are schematic diagrams illustrating patterns of combinations of four electrodes in the seventh arrangement example.
[FIG. 11] FIG. 11 is a flowchart illustrating an operation of the distribution measurement device according to the first embodiment.
[FIG. 12] FIG. 12 is a fluid circuit diagram of a distribution measurement device according to a second embodiment.
[FIG. 13] FIG. 13 is a block diagram of the distribution measurement device according to the second embodiment.
[FIG. 14] FIG. 14 is a flowchart illustrating an operation of the distribution measurement device according to the second embodiment.
[FIG. 15] FIG. 15 is a block diagram of a distribution measurement device according to a third embodiment.
[FIG. 16] FIGS. 16A and 16B are schematic diagrams illustrating arrangements of a plurality of electrodes in the third embodiment.
[FIG. 17] FIG. 17 is a flowchart illustrating an operation of the distribution measurement device according to the third embodiment.
[FIG. 18] FIG. 18 is a schematic diagram illustrating an arrangement of a plurality of electrodes in a fourth embodiment.
[FIG. 19] FIGS. 19A and 19B are schematic diagrams illustrating arrangements of a plurality of electrodes in a fifth embodiment.

### Description of Embodiments

### [1 First Embodiment]

### [1-1 Configuration of Distribution Measurement Device 10]

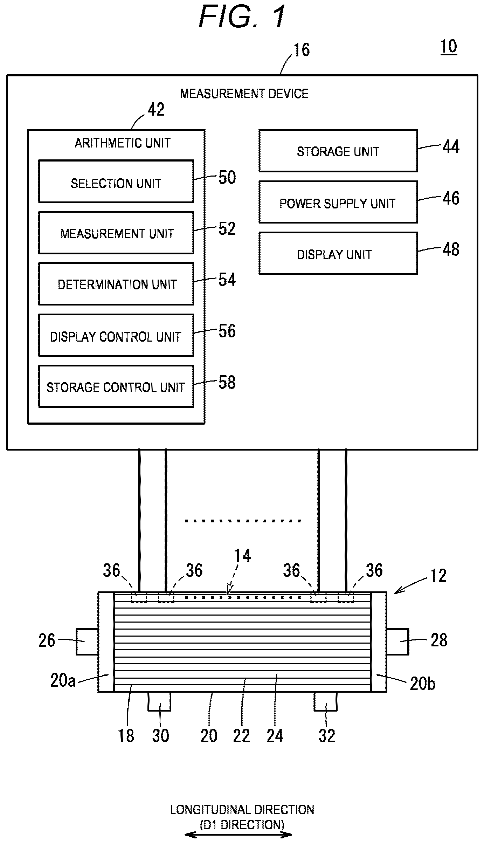

FIG. 1 is a block diagram of a distribution measurement device 10 according to the first embodiment. The distribution measurement device 10 according to the first embodiment can measure a distribution of cells in a longitudinal direction of a bioreactor 12. Examples of the cells include ES cells, iPS cells, and mesenchymal stem cells. Note that the cells are not limited to the cells described above, and may be yeast or the like. The distribution measurement device 10 includes the bioreactor 12, an electrode group 14, and a measurement device 16.

The bioreactor 12 is a component of a cell culture device (not illustrated). The bioreactor 12 includes a plurality of hollow fiber membranes 18 and a cylindrical housing 20. The plurality of hollow fiber membranes 18 are housed inside the housing 20. A longitudinal direction of each of the hollow fiber membranes 18 is along the longitudinal direction of the bioreactor 12. That is, the hollow fiber membrane 18 extends along the longitudinal direction of the bioreactor 12. The hollow fiber membrane 18 is made of, for example, a polymer material. The hollow fiber membrane 18 has a plurality of pores (not illustrated) . A first end portion of the hollow fiber membrane 18 is fixed to a first end portion 20a of the housing 20 in the longitudinal direction. A second end portion of the hollow fiber membrane 18 is fixed to a second end portion 20b of the housing 20 in the longitudinal direction.

The bioreactor 12 includes a first region 22 and a second region 24. The first region 22 is a space inside the hollow fiber membrane 18. The second region 24 is a space between an outer peripheral surface of the hollow fiber membrane 18 and an inner peripheral surface of the housing 20. The first region 22 and the second region 24 communicate with each other via a plurality of pores of the hollow fiber membranes 18.

The housing 20 includes a first port 26 (first supply and discharge port), a second port 28 (second supply and discharge port), a third port 30, and a fourth port 32. The first port 26 is disposed at the first end portion 20a of the housing 20. The first port 26 is connected to the first end portion of each of the hollow fiber membranes 18. Accordingly, the first port 26 communicates with the first region 22. The second port 28 is disposed at the second end portion 20b of the housing 20. The second port 28 is connected to the second end portion of each of the hollow fiber membranes 18. Accordingly, the second port 28 communicates with the first region 22.

The third port 30 and the fourth port 32 are disposed on an outer peripheral surface of the housing 20. The third port 30 is disposed between the first port 26 and a central portion of the housing 20 in the longitudinal direction. The fourth port 32 is disposed between the second port 28 and the central portion of the housing 20 in the longitudinal direction. Each of the third port 30 and the fourth port 32 communicates with the second region 24.

A cell suspension containing cells is supplied from the first port 26 or the second port 28 to an inside (first region 22) of the bioreactor 12. A culture medium is supplied from the first port 26 or the second port 28 to the inside (first region 22) of the bioreactor 12. The culture medium is supplied from the third port 30 or the fourth port 32 to the inside (second region 24) of the bioreactor 12. The culture medium supplied to the bioreactor 12 can move between the first region 22 and the second region 24 via pores of the hollow fiber membrane 18.

The electrode group 14 includes a plurality of (three or more) electrodes 36. The plurality of electrodes 36 are disposed inside the bioreactor 12 along the longitudinal direction of the bioreactor 12. An arrangement of the plurality of electrodes 36 will be described later.

The measurement device 16 can measure electrostatic capacitance (or dielectric constant, hereinafter omitted) between a pair of electrodes 36. The electrostatic capacitance between the pair of electrodes 36 is a physical quantity proportional to a cell concentration (the number of cells, hereinafter omitted) between the pair of electrodes 36. The measurement device 16 may be, for example, an impedance analyzer or an LCR meter. The measurement device 16 includes an arithmetic unit 42, a storage unit 44, a power supply unit 46, and a display unit 48.

The arithmetic unit 42 may be implemented by, for example, a processor such as a central processing unit (CPU) or a graphics processing unit (GPU). That is, the arithmetic unit 42 may be implemented by a processing circuit.

The arithmetic unit 42 includes a selection unit 50, a measurement unit 52, a determination unit 54, a display control unit 56, and a storage control unit 58. Each of the selection unit 50, the measurement unit 52, the determination unit 54, the display control unit 56, and the storage control unit 58 can be implemented by the arithmetic unit 42 executing a program stored in the storage unit 44.

Note that at least a part of the selection unit 50, the measurement unit 52, the determination unit 54, the display control unit 56, and the storage control unit 58 may be implemented by an integrated circuit such as an application specific integrated circuit (ASIC) or a field-programmable gate array (FPGA). At least a part of the selection unit 50, the measurement unit 52, the determination unit 54, the display control unit 56, and the storage control unit 58 may be implemented by an electronic circuit including a discrete device.

The selection unit 50 sequentially selects a pair of electrodes 36 from the plurality of electrodes 36 (electrode group 14). The measurement unit 52 executes switching control of the electrode 36 based on a selection result of the selection unit 50. Further, the measurement unit 52 sequentially measures electrostatic capacitance between the pair of electrodes 36 selected by the selection unit 50. The determination unit 54 performs various determinations. The display control unit 56 executes display control. For example, the display control unit 56 displays, on the display unit 48, information indicating a distribution of cells in the longitudinal direction of the bioreactor 12 based on the electrostatic capacitance measured by the measurement unit 52. The storage control unit 58 executes storage control. For example, the storage control unit 58 stores, in the storage unit 44, information indicating the distribution of cells in the longitudinal direction of the bioreactor 12 based on the electrostatic capacitance measured by the measurement unit 52.

The storage unit 44 may include a volatile memory (not illustrated) and a non-volatile memory (not illustrated). Examples of the volatile memory include a random access memory (RAM). The volatile memory is used as a working memory of the processor and temporarily stores data and the like necessary for processing or calculation. Examples of the non-volatile memory include a read only memory (ROM) and a flash memory. The non-volatile memory is used as a memory for storage and stores programs, tables, maps, and the like. At least a part of the storage unit 44 may be in the processor, the integrated circuit, or the like as described above.

The storage unit 44 can store information indicating the distribution of cells in the longitudinal direction of the bioreactor 12 according to the storage control executed by the storage control unit 58 of the arithmetic unit 42. Note that a part of the storage unit 44 may be provided outside the measurement device 16.

The power supply unit 46 includes a power supply circuit capable of applying a voltage (or current) between the pair of electrodes 36. The power supply circuit includes a plurality of switches. Each of the switches switches connection and disconnection between a power supply (not illustrated) and the electrode 36 according to a switching signal output from the arithmetic unit 42 (measurement unit 52).

The display unit 48 includes a drive circuit and a display. The display unit 48 can display information indicating the distribution of cells in the longitudinal direction of the bioreactor 12 according to the display control executed by the arithmetic unit 42 (display control unit 56). For example, the display unit 48 may display the cell concentration in a numerical value. Alternatively, the display unit 48 may display the cell concentration as a graph.

### [1-2 Arrangement of Plurality of Electrodes 36]

The arrangement of the plurality of electrodes 36 will be described below. Note that in the following description, the longitudinal direction of the bioreactor 12 is also referred to as a D1 direction. Among radial directions of the bioreactor 12, two directions that are perpendicular to each other are referred to as a D2 direction and a D3 direction. The D2 direction and the D3 direction are perpendicular to the D1 direction.

### [A First Arrangement Example]

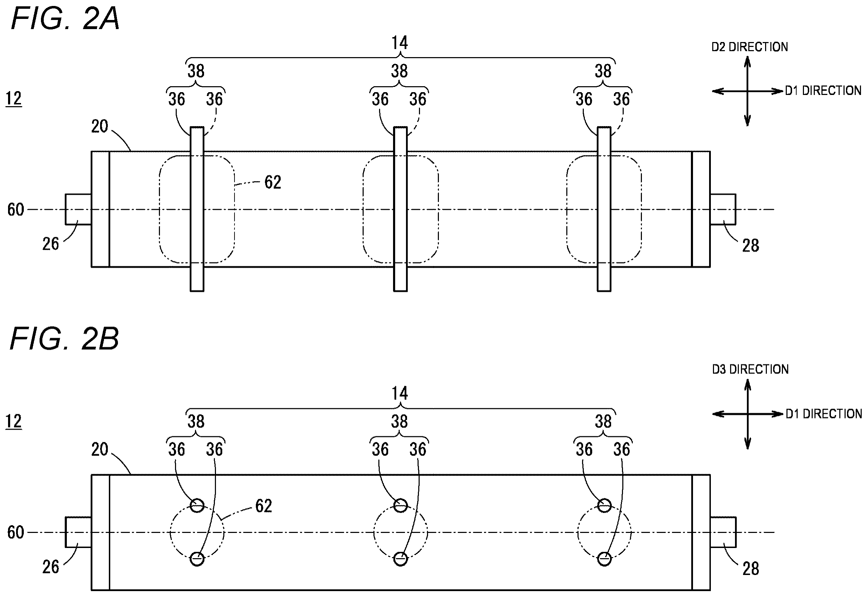

FIGS. 2A and 2B are schematic diagrams illustrating arrangements of the plurality of electrodes 36 in the first arrangement example. FIG. 2A illustrates a position relationship between the bioreactor 12 and the plurality of electrodes 36 in a plan view of the bioreactor 12. FIG. 2B illustrates a position relationship between the bioreactor 12 and the plurality of electrodes 36 in a front view of the bioreactor 12.

As illustrated in FIG. 2A, each of the electrodes 36 extend along the D2 direction. That is, the electrode 36 extends along a direction intersecting the D1 direction. The electrode 36 penetrates the housing 20 of the bioreactor 12. The electrode 36 is insulated with respect to the housing 20. The electrode 36 is connected to the power supply unit 46 (FIG. 1).

As illustrated in FIG. 2B, two electrodes 36 of the plurality of electrodes 36 are disposed along the D3 direction. These two electrodes 36 are spaced apart from each other to sandwich an axis 60 of the housing 20 (bioreactor 12). When the electrostatic capacitance is measured, these two electrodes 36 are simultaneously selected. One of the two electrodes 36 is a positive electrode, and the other is a negative electrode. That is, when the electrostatic capacitance is measured, these two electrodes 36 form a pair. In this way, two electrodes 36 that form a pair in which only one electrode 36 is correlated with one electrode 36 in advance are referred to as an "assembled electrode 38". The electrode group 14 includes a plurality of assembled electrodes 38. The plurality of assembled electrodes 38 are spaced apart from each other along the D1 direction. For example, the assembled electrodes 38 are disposed at equal intervals along the D1 direction.

When the power supply unit 46 (FIG. 1) applies a voltage (or a current) to the assembled electrode 38 (the pair of electrodes 36), the assembled electrode 38 stores a charge corresponding to a cell concentration between the electrodes 36. Note that the cell concentration is substantially constant in a certain range centered on the assembled electrode 38. From the above, the assembled electrode 38 functions as a sensor member for measuring the cell concentration in a certain range. This certain range is referred to as a measurement range 62. The measurement range 62 is determined by a distance between the positive electrode and the negative electrode of the assembled electrode 38 (inter-electrode distance) and a length of each electrode 36. The length of the electrode 36 and the inter-electrode distance are preferably set such that the measurement range 62 is wide. From a viewpoint of reducing the number of electrodes 36, the length of the electrode 36 and the inter-electrode distance are preferably set such that the measurement range 62 is maximized. Further, the plurality of assembled electrodes 38 are preferably disposed along the D1 direction such that two measurement ranges 62 adjacent to each other are as close as possible. When these conditions are satisfied, the distribution of the cell concentration in the D1 direction in the bioreactor 12 can be measured with the minimum number of assembled electrodes 38. These conditions are not essential.

### [B Second Arrangement Example]

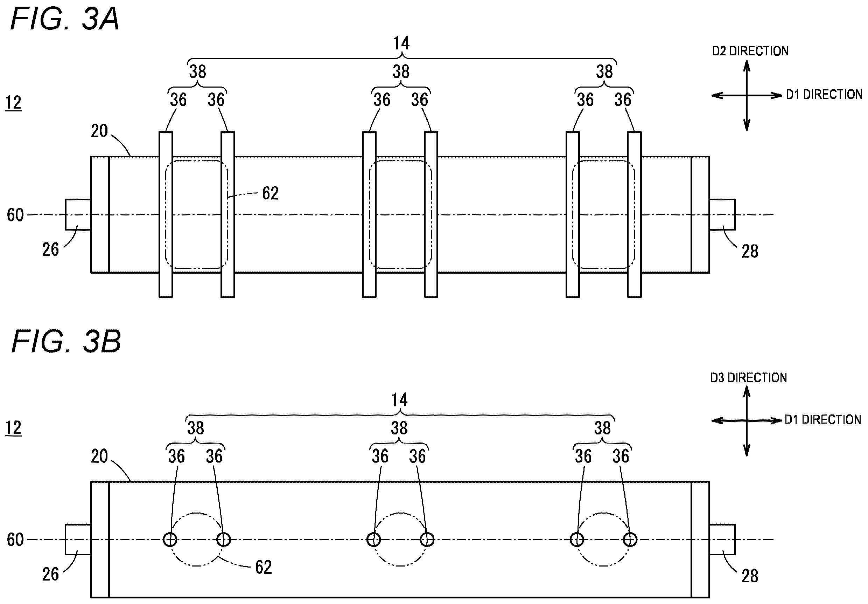

FIGS. 3A and 3B are schematic diagrams illustrating arrangements of the plurality of electrodes 36 in the second arrangement example. FIG. 3A illustrates a position relationship between the bioreactor 12 and the plurality of electrodes 36 in the plan view of the bioreactor 12. FIG. 3B illustrates a position relationship between the bioreactor 12 and the plurality of electrodes 36 in the front view of the bioreactor 12. Regarding the second arrangement example, portions different from the first arrangement example will be described. The second arrangement example is different from the first arrangement example in a disposition direction of the two electrodes 36 in one assembled electrode 38.

As illustrated in FIG. 3B, the plurality of electrodes 36 are disposed along the D1 direction. Preferably, all the electrodes 36 intersect the axis 60 of the housing 20. For example, all the electrodes 36 are perpendicular to the axis 60 of the housing 20. All electrodes 36 or some of the electrodes 36 may deviate from the axis 60. The electrode 36 forms an assembled electrode 38 (positive electrode and negative electrode) with the most adjacent electrode 36. In this way, the electrode group 14 includes a plurality of assembled electrodes 38. The plurality of assembled electrodes 38 are spaced apart from each other along the D1 direction. For example, the assembled electrodes 38 are disposed at equal intervals along the D1 direction.

### [C Third Arrangement Example]

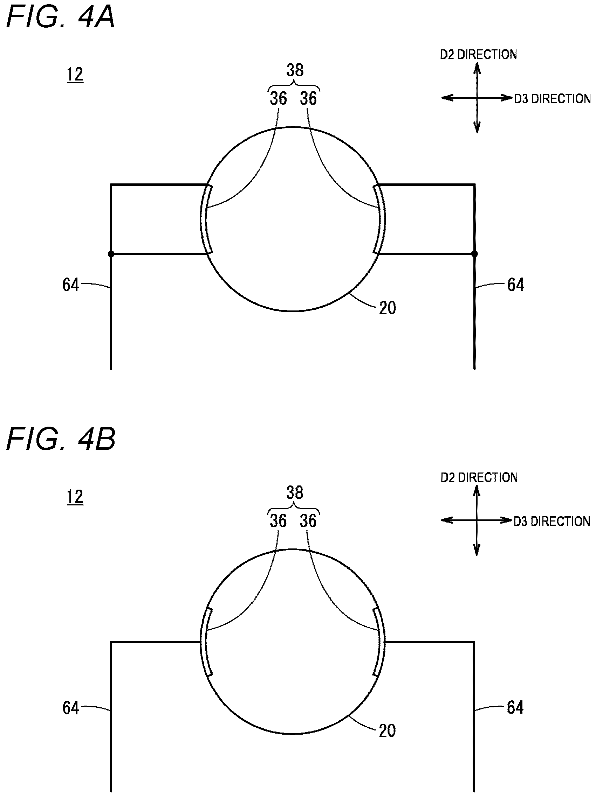

FIGS. 4A and 4B are schematic diagrams illustrating an arrangement of one assembled electrode 38 in the third arrangement example. FIGS. 4A and 4B illustrate a position relationship between the bioreactor 12 and one assembled electrode 38 in a side view of the bioreactor 12. The third arrangement example is a modification of the first arrangement example. Regarding the third arrangement example, portions different from the first arrangement example will be described. The third arrangement example is different from the first arrangement example in a shape of the two electrodes 36 forming one assembled electrode 38.

As illustrated in FIG. 4A, the electrode 36 is curved along the inner peripheral surface of the housing 20 of the bioreactor 12. The electrode 36 is attached to the inner peripheral surface of the housing 20. The electrode 36 is insulated with respect to the housing 20. The electrode 36 is connected to the power supply unit 46 (FIG. 1) via a lead wire 64. As illustrated in FIG. 4A, the lead wire 64 may be drawn out from both end portions of the electrode 36. As illustrated in FIG. 4B, the lead wire 64 may be drawn out from a part of the electrode 36.

### [D Fourth Arrangement Example]

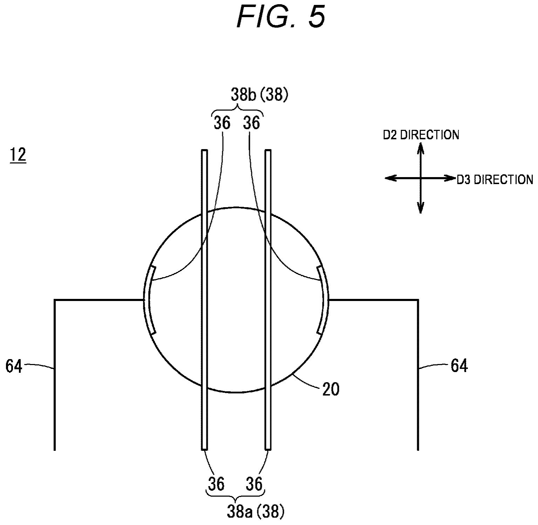

FIG. 5 is a schematic diagram illustrating an arrangement of one assembled electrode 38 in the fourth arrangement example. The fourth arrangement example is a modification of the first arrangement example and the third arrangement example. The assembled electrode 38 in the fourth arrangement example includes the assembled electrode 38 in the first arrangement example (hereinafter referred to as a first assembled electrode 38a) and the assembled electrode 38 in the third arrangement example (hereinafter referred to as a third assembled electrode 38b). The first assembled electrode 38a is disposed between the electrodes of the third assembled electrode 38b.

In the fourth arrangement example, the power supply unit 46 applies a voltage (or current) to the third assembled electrode 38b and measures the electrostatic capacitance of the first assembled electrode 38a. According to the fourth arrangement example, an influence of an electric double layer can be reduced.

### [E Fifth Arrangement Example]

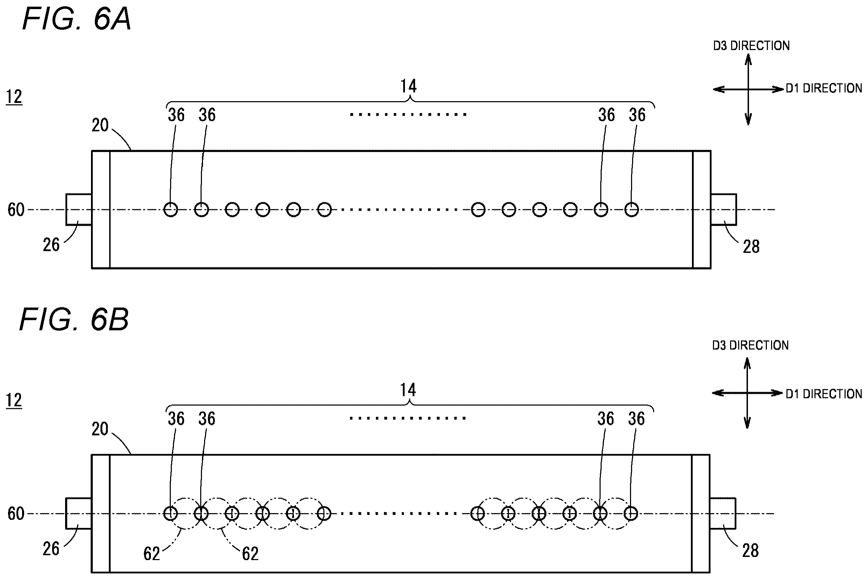

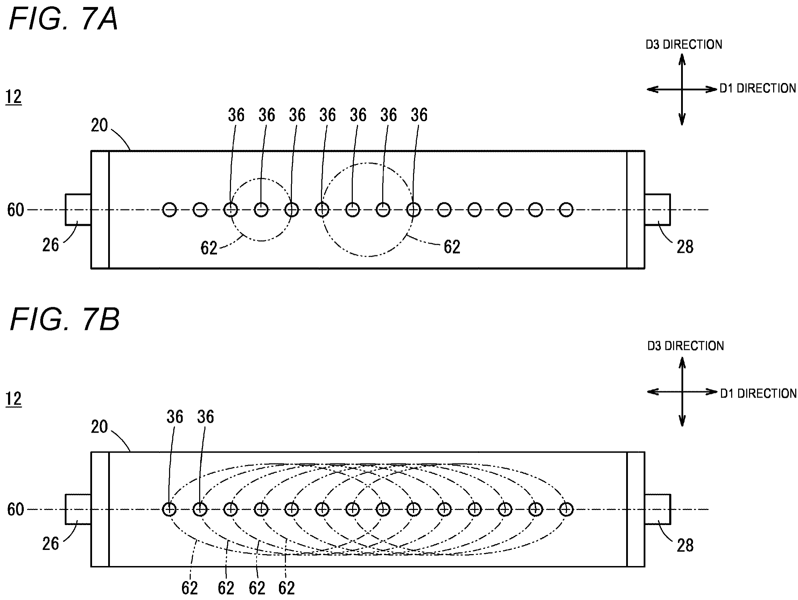

FIGS. 6A, 6B, 7A, and 7B are schematic diagrams illustrating arrangements of the plurality of electrodes 36 in the fifth arrangement example. The fifth arrangement example is a modification of the second arrangement example. As illustrated in FIG. 6A, in the fifth arrangement example, the plurality of electrodes 36 are disposed at equal intervals along the D1 direction. In the case of the fifth arrangement example, the selection unit 50 (FIG. 1) can select any two electrodes 36.

As illustrated in FIG. 6B, the selection unit 50 may sequentially select two electrodes 36 adjacent to each other as a pair of electrodes 36. As illustrated in FIG. 7A, the selection unit 50 may sequentially select two electrodes 36 sandwiching one or more electrodes 36 as a pair of electrodes 36. As illustrated in FIG. 7B, the selection unit 50 may sequentially select the pair of electrodes 36 such that a plurality of measurement ranges 62 overlap each other. According to the fifth arrangement example, the measurement range 62 can be expanded in the D1 direction, the D2 direction, and the D3 direction.

### [F Sixth Arrangement Example]

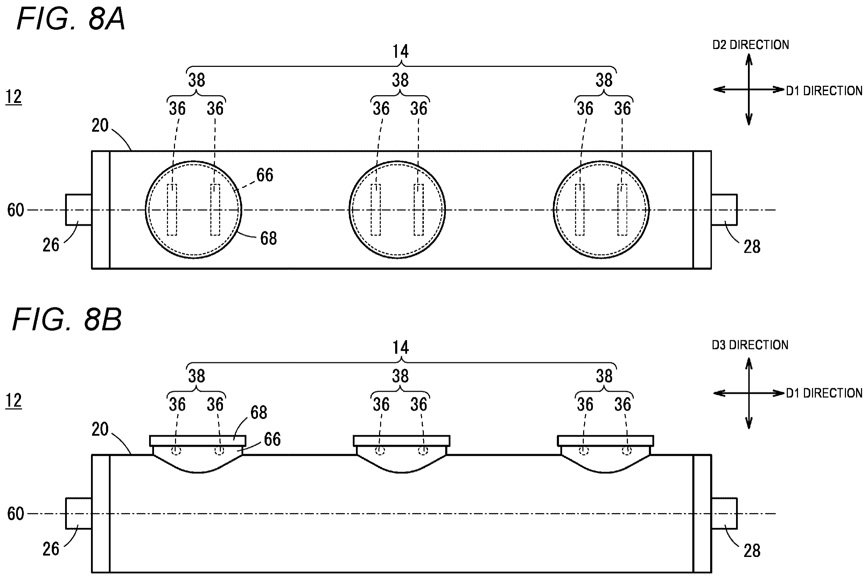

FIGS. 8A and 8B are schematic diagrams illustrating arrangements of the plurality of electrodes 36 in the sixth arrangement example. FIG. 8A illustrates a position relationship between the bioreactor 12 and the plurality of electrodes 36 in the plan view of the bioreactor 12. FIG. 8B illustrates a position relationship between the bioreactor 12 and the plurality of electrodes 36 in the front view of the bioreactor 12.

The housing 20 of the bioreactor 12 includes a plurality of convex portions 66. Each of the plurality of convex portions 66 protrudes in the same direction (D3 direction) from the outer peripheral surface (for example, an upper surface) of the housing 20. The plurality of convex portions 66 are spaced apart from each other along the D1 direction. Each of the convex portions 66 is hollow. A lid portion 68 is attachable to and detachable from a tip of the convex portion 66. The lid portion 68 is parallel to the D2 direction and perpendicular to the D3 direction when attached to the convex portion 66. The lid portion 68 has two electrodes 36 disposed thereon. The two electrodes 36 are parallel to each other. For example, each of the electrodes 36 extends along the D2 direction. The two electrodes 36 disposed on the lid portion 68 form an assembled electrode 38.

### [G Seventh Arrangement Example]

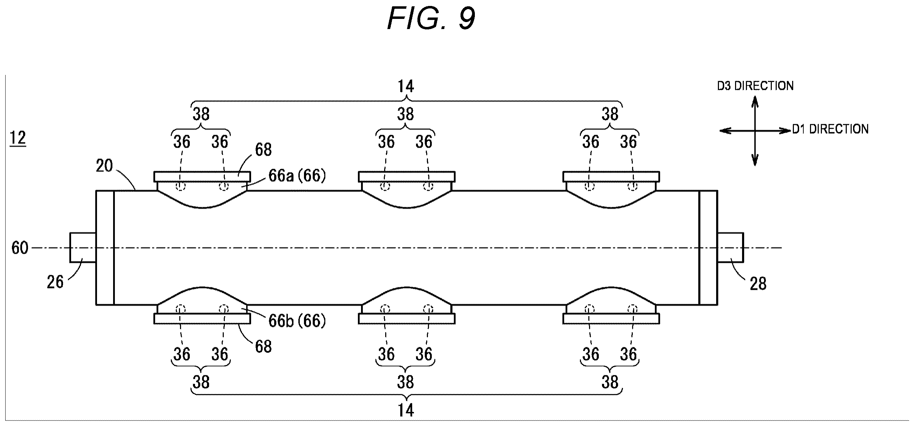

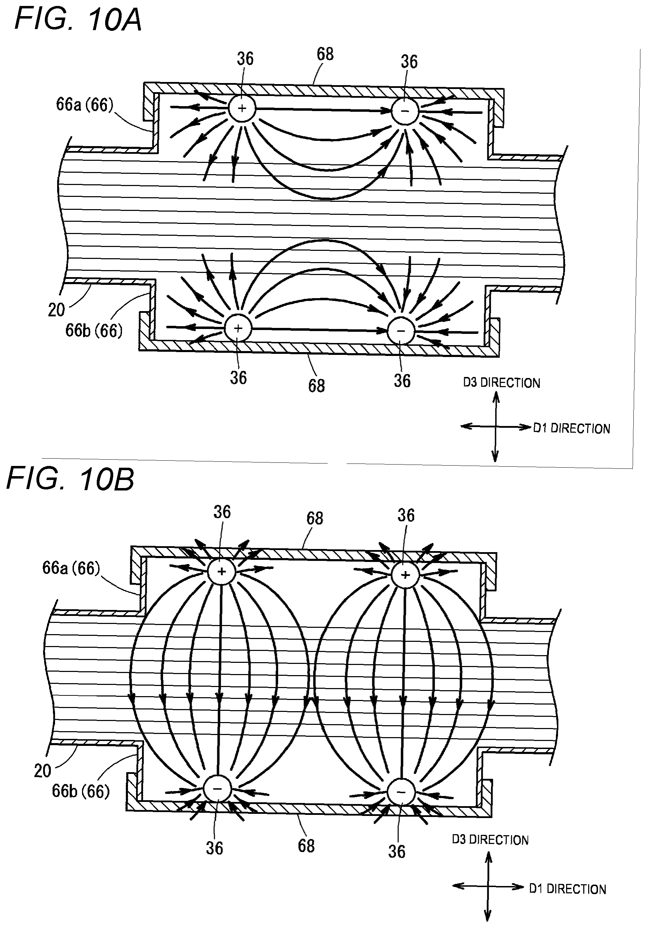

FIG. 9 is a schematic diagram illustrating an arrangement of the plurality of electrodes 36 in the seventh arrangement example. FIG. 9 illustrates a position relationship between the bioreactor 12 and the plurality of electrodes 36 in the front view of the bioreactor 12. FIGS. 10A and 10B are schematic diagrams illustrating patterns of combinations of four electrodes 36 in the seventh arrangement example.

As in the sixth arrangement example, in the seventh arrangement example, the housing 20 of the bioreactor 12 includes a plurality of convex portions 66. A structure of the convex portion 66 according to the seventh arrangement example is the same as a structure of the convex portion 66 according to the sixth arrangement example.

In the seventh arrangement example, the plurality of convex portions 66 are divided into two groups. Each of the convex portions 66 in a first group (hereinafter referred to as a first convex portion 66a) protrudes from the upper surface of the housing 20 along the D3 direction. Each of the convex portions 66 in a second group (hereinafter referred to as a second convex portion 66b) protrudes from a lower surface of the housing 20 along the D3 direction. One first convex portion 66a and one second convex portion 66b are disposed along the D3 direction. Further, one of the two electrodes 36 in the first convex portion 66a and one of the two electrodes 36 in the second convex portion 66b are disposed along the D3 direction. Similarly, the other of the two electrodes 36 in the first convex portion 66a and the other of the two electrodes 36 in the second convex portion 66b are disposed along the D3 direction.

As illustrated in FIG. 10A, the selection unit 50 may select the two electrodes 36 disposed along the D1 direction as a pair of electrodes 36. As illustrated in FIG. 10B, the selection unit 50 may select two electrodes 36 disposed along the D3 direction as a pair of electrodes 36.

### [1-3 Operation of Distribution Measurement Device 10]

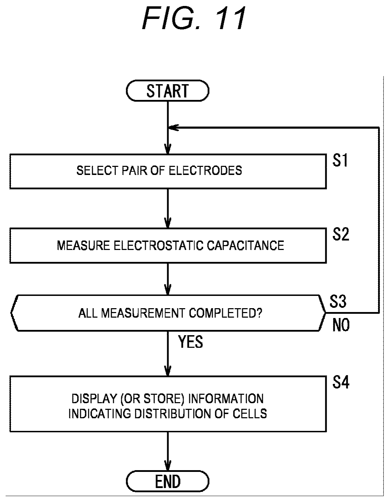

FIG. 11 is a flowchart illustrating the operation of the distribution measurement device 10 (FIG. 1) according to the first embodiment.

In step S1, the selection unit 50 selects a pair of electrodes 36 from the plurality of electrodes 36. For example, as illustrated in FIG. 2A and the like, when the assembled electrode 38 is formed in advance, the selection unit 50 selects the assembled electrode 38 for which measurement of the electrostatic capacitance is not completed.

In step S2, the measurement unit 52 executes switching control of the power supply unit 46 so that power is supplied to the pair of electrodes 36 selected by the selection unit 50. The power supply unit 46 performs switching according to a switching signal output from the measurement unit 52, and applies a voltage between the pair of electrodes 36. The measurement unit 52 measures electrostatic capacitance between the selected pair of electrodes 36.

In step S3, the determination unit 54 determines whether the measurement of all the electrostatic capacitance is completed. If the measurement of all the electrostatic capacitance is completed (step S3: YES), the processing proceeds to step S4. Meanwhile, if the measurement of some of the electrostatic capacitance is not completed (step S3: NO), the processing returns to step S1.

When the processing proceeds from step S3 to step S4, the measurement unit 52 converts the electrostatic capacitance into a cell concentration using a calibration curve that indicates a relationship between the electrostatic capacitance and the cell concentration. The storage unit 44 stores the calibration curve in advance. The display control unit 56 executes display control for displaying information indicating a distribution of cells in the longitudinal direction (D1 direction) of the bioreactor 12. The display unit 48 displays information indicating the distribution of cells according to the display control. For example, the display unit 48 may display a graph or a numerical value as the information indicating the distribution of cells. Meanwhile, the storage control unit 58 stores, in the storage unit 44, information indicating the distribution of cells in the longitudinal direction (D1 direction) of the bioreactor 12.

As described above, in the first embodiment, the plurality of pairs of electrodes 36 form the plurality of cell concentration measurement ranges 62 along the D1 direction in the bioreactor 12. Therefore, according to the first embodiment, the distribution of cells in the longitudinal direction of the bioreactor 12 can be measured. The distribution of cells in the longitudinal direction of the bioreactor 12 contributes to optimization of cell culture.

### [2 Second Embodiment]

### [2-1 Configuration of Distribution Measurement Device 10]

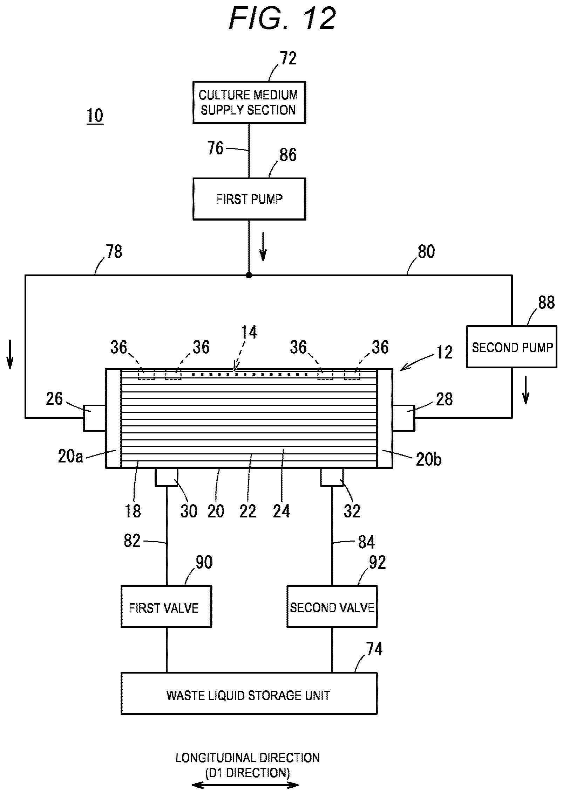

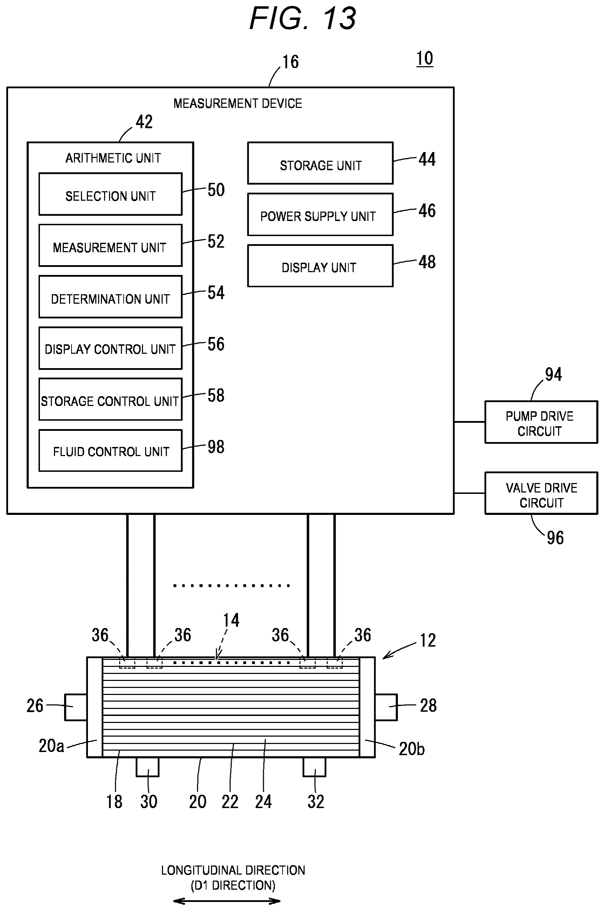

FIG. 12 is a fluid circuit diagram of the distribution measurement device 10 according to the second embodiment. FIG. 13 is a block diagram of the distribution measurement device 10 according to the second embodiment. The distribution measurement device 10 according to the second embodiment can measure a distribution of cells in a longitudinal direction of the bioreactor 12. Further, when the cells to be cultured are floating cells, the distribution measurement device 10 according to the second embodiment can change the distribution of the cells in the longitudinal direction of the bioreactor 12.

The distribution measurement device 10 according to the second embodiment may be incorporated in a cell culture device that cultures cells. In this case, some components of the cell culture device may be used as components of the distribution measurement device 10. Note that in the following description, components having the same functions as those of the first embodiment are denoted by the same reference signs, and description thereof will be omitted.

As illustrated in FIG. 12, the distribution measurement device 10 includes a culture medium supply section 72 and a waste liquid storage unit 74. The culture medium supply section 72 includes a medical bag filled with a culture medium. The waste liquid storage unit 74 includes a medical bag capable of collecting waste liquid discharged from the bioreactor 12.

The distribution measurement device 10 includes a supply flow path 76, a first branch flow path 78, a second branch flow path 80, a first waste liquid flow path 82, and a second waste liquid flow path 84. Each of the flow paths includes a tube through which liquid flows. The supply flow path 76 is connected to the culture medium supply section 72, the first branch flow path 78, and the second branch flow path 80. The first branch flow path 78 is connected to the supply flow path 76 and the first port 26 of the bioreactor 12. The second branch flow path 80 is connected to the supply flow path 76 and the second port 28 of the bioreactor 12. The first waste liquid flow path 82 is connected to the third port 30 of the bioreactor 12 and the waste liquid storage unit 74. The second waste liquid flow path 84 is connected to the fourth port 32 of the bioreactor 12 and the waste liquid storage unit 74.

The distribution measurement device 10 includes a first pump 86 and a second pump 88. The first pump 86 is disposed in the supply flow path 76. The second pump 88 is disposed in the second branch flow path 80. The first pump 86 can apply a flow force to a culture medium in the supply flow path 76 in a direction toward the first branch flow path 78 and the second branch flow path 80. The second pump 88 can apply a flow force to a culture medium in the second branch flow path 80 in a direction toward the second port 28 or a direction toward the first port 26.

The distribution measurement device 10 includes a first valve 90 and a second valve 92. The first valve 90 is disposed in the first waste liquid flow path 82. The second valve 92 is disposed in the second waste liquid flow path 84. The first valve 90 and the second valve 92 each include a clamp capable of opening and closing the flow path (tube).

As illustrated in FIG. 13, the arithmetic unit 42 includes a fluid control unit 98 (control unit). The fluid control unit 98 can be implemented by the arithmetic unit 42 executing a program stored in the storage unit 44.

The fluid control unit 98 outputs various operation signals and controls an operation of the first pump 86, an operation of the second pump 88, an operation of the first valve 90, and an operation of the second valve 92. The fluid control unit 98 can control at least one of an inflow rate of a culture medium into the bioreactor 12 and an outflow rate of the culture medium from the bioreactor 12.

The distribution measurement device 10 includes a pump drive circuit 94 and a valve drive circuit 96. The pump drive circuit 94 supplies power to the first pump 86 and the second pump 88 according to an operation signal output from the measurement device 16 (fluid control unit 98). The valve drive circuit 96 supplies power to the first valve 90 and the second valve 92 according to an operation signal output from the measurement device 16 (fluid control unit 98).

### [2-2 Operation of Distribution Measurement Device 10]

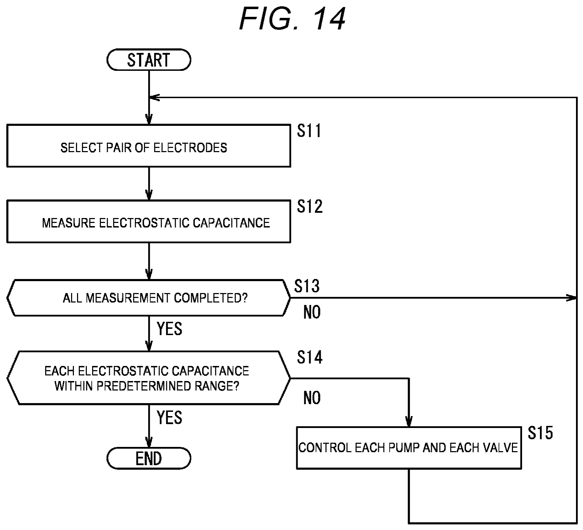

FIG. 14 is a flowchart illustrating the operation of the distribution measurement device 10 according to the second embodiment. A series of processes illustrated in FIG. 14 may be in a cell culture processing. For example, in the cell culture processing, a cell agitation step of agitating cells inside the bioreactor 12, a cell packing step of packing cells remaining in the flow path into the bioreactor 12, and a cell culture step of culturing the cells are repeatedly performed.

The series of processes illustrated in FIG. 14 may be performed in the cell packing step. As indicated by arrows in FIG. 12, in the cell packing step, the culture medium is supplied from the culture medium supply section 72 to the bioreactor 12 via the supply flow path 76 and the first branch flow path 78. At the same time, in the cell packing step, the culture medium is supplied from the culture medium supply section 72 to the bioreactor 12 via the supply flow path 76 and the second branch flow path 80. Accordingly, the cells remaining in the first branch flow path 78 and the second branch flow path 80 are packed into the bioreactor 12.

Step S11 to step S13 illustrated in FIG. 14 are the same as step S1 to step S3 illustrated in FIG. 11. Therefore, the description of step S11 to step S13 will be omitted here.

In step S13, if the measurement of all the electrostatic capacitance is completed (step S13: YES), the processing proceeds to step S14. In step S14, the determination unit 54 determines whether each measured electrostatic capacitance is within a predetermined range. The predetermined range is set based on the cell concentration in a state where the cells are uniformly diffused inside the bioreactor 12. A cell concentration in a state where cells are uniformly diffused is referred to as a reference value. For example, the predetermined range is set to a range between an upper limit value larger than the reference value by a predetermined value and a lower limit value smaller than the reference value by a predetermined value. The storage unit 44 stores the predetermined range in advance. If each electrostatic capacitance is within the predetermined range (step S14: YES), the series of processes illustrated in FIG. 14 ends. In this case, the cell concentration at each position along the longitudinal direction of the bioreactor 12 is substantially uniform. Meanwhile, if at least one electrostatic capacitance is out of the predetermined range (step S14: NO), the processing proceeds to step S15. In this case, the cell concentration at each position along the longitudinal direction of the bioreactor 12 is nonuniform.

When the processing proceeds from step S14 to step S15, the fluid control unit 98 controls each pump and each valve such that the cell concentration at positions along the longitudinal direction of the bioreactor 12 becomes uniform.

For example, a cell concentration at a position close to the first port 26 may be higher than a cell concentration at a position close to the second port 28. In this case, the fluid control unit 98 controls the first pump 86 and the second pump 88 such that an inflow rate of the culture medium into the first port 26 is higher than an inflow rate of the culture medium into the second port 28. In this case, a flow force from the first port 26 toward the second port 28 is larger than a flow force from the second port 28 toward the first port 26 inside the bioreactor 12. Further, the fluid control unit 98 closes the first valve 90 and opens the second valve 92. Then, inside the bioreactor 12, some of the cells diffusing near the first port 26 move toward the second port 28. As a result, a bias of the cell concentration inside the bioreactor 12 is corrected. The excess culture medium inside the bioreactor 12 is discharged to the waste liquid storage unit 74 via the second waste liquid flow path 84.

Meanwhile, a cell concentration at a position close to the second port 28 may be higher than a cell concentration at a position close to the first port 26. In this case, the fluid control unit 98 controls the first pump 86 and the second pump 88 such that an inflow rate of the culture medium into the second port 28 is higher than an inflow rate of the culture medium into the first port 26. In this case, a flow force from the second port 28 toward the first port 26 is larger than a flow force from the first port 26 toward the second port 28 inside the bioreactor 12. Further, the fluid control unit 98 closes the second valve 92 and opens the first valve 90. Then, inside the bioreactor 12, some of the cells diffusing near the second port 28 move toward the first port 26. As a result, a bias of the cell concentration inside the bioreactor 12 is corrected. The excess culture medium inside the bioreactor 12 is discharged to the waste liquid storage unit 74 via the first waste liquid flow path 82.

When a predetermined condition is satisfied after the processing of step S15 is started, the processing of step S11 and subsequent steps is executed again. For example, when a supply amount of the culture medium in step S15 exceeds a predetermined amount, or when an execution time in step S15 exceeds a predetermined time, the processing of step S11 and subsequent steps is executed again.

As described above, in the second embodiment, the fluid control unit 98 controls the pump and the valve based on the distribution of cells such that the distribution of cells in the longitudinal direction of the bioreactor 12 becomes uniform. When the distribution of the cells in the longitudinal direction of the bioreactor 12 is uniform, nutrition is uniformly distributed to the cells inside the bioreactor 12. Therefore, according to the second embodiment, it is possible to contribute to optimization of cell culture.

### [3 Third Embodiment]

### [3-1 Configuration of Distribution Measurement Device 10]

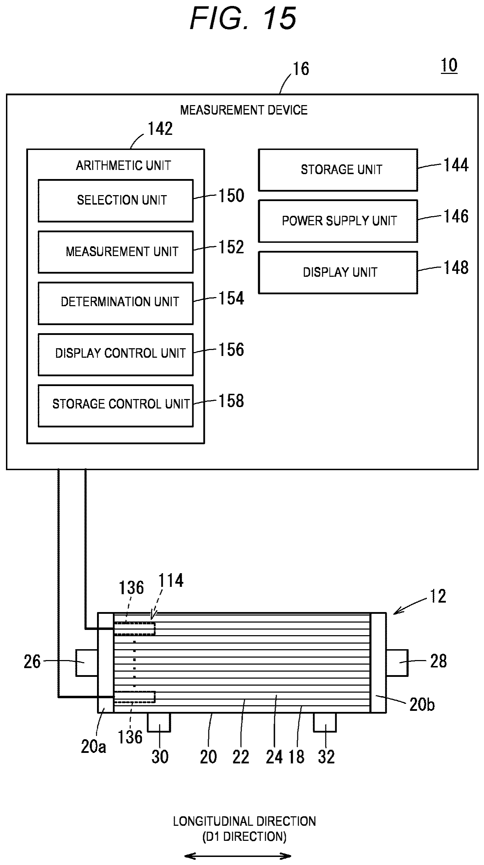

FIG. 15 is a block diagram of the distribution measurement device 10 according to the third embodiment. The distribution measurement device 10 according to the third embodiment can measure a distribution of cells in a direction intersecting a longitudinal direction of the bioreactor 12. Examples of the cells include ES cells, iPS cells, and mesenchymal stem cells. Note that the cells are not limited to the cells described above, and may be yeast or the like. The distribution measurement device 10 includes the bioreactor 12, an electrode group 114, and the measurement device 16.

In the description of the third embodiment (and a fourth embodiment and a fifth embodiment), the same components as those of the first embodiment and the second embodiment are denoted by the same reference signs, and the description thereof will be omitted.

The electrode group 114 includes a plurality of (three or more) electrodes 136. Each of the electrode 136 is disposed inside the bioreactor 12. A longitudinal direction of the electrode 136 is along the longitudinal direction of the bioreactor 12. That is, the electrode 136 extends along the longitudinal direction of the bioreactor 12. An arrangement of the plurality of electrodes 136 will be described later.

The measurement device 16 can measure electrostatic capacitance (or dielectric constant, hereinafter omitted) between a pair of electrodes 136. The electrostatic capacitance between the pair of electrodes 136 is a physical quantity proportional to a cell concentration (the number of cells, hereinafter omitted) between the pair of electrodes 136. The measurement device 16 may be, for example, an impedance analyzer or an LCR meter. The measurement device 16 includes an arithmetic unit 142, a storage unit 144, a power supply unit 146, and a display unit 148.

The arithmetic unit 142 may be implemented by, for example, a processor such as a central processing unit (CPU) or a graphics processing unit (GPU). That is, the arithmetic unit 142 may be implemented by a processing circuit.

The arithmetic unit 142 includes a selection unit 150, a measurement unit 152, a determination unit 154, a display control unit 156, and a storage control unit 158. Each of the selection unit 150, the measurement unit 152, the determination unit 154, the display control unit 156, and the storage control unit 158 can be implemented by the arithmetic unit 142 executing a program stored in the storage unit 144.

Note that at least a part of the selection unit 150, the measurement unit 152, the determination unit 154, the display control unit 156, and the storage control unit 158 may be implemented by an integrated circuit such as an application specific integrated circuit (ASIC) or a field-programmable gate array (FPGA). At least a part of the selection unit 150, the measurement unit 152, the determination unit 154, the display control unit 156, and the storage control unit 158 may be implemented by an electronic circuit including a discrete device.

The selection unit 150 sequentially selects a pair of electrodes 136 from the plurality of electrodes 136 (electrode group 114). The measurement unit 152 executes switching control of the electrode 136 based on a selection result of the selection unit 150. Further, the measurement unit 152 sequentially measures electrostatic capacitance between the pair of electrodes 136 selected by the selection unit 150. The determination unit 154 performs various determinations. The display control unit 156 executes display control. For example, the display control unit 156 displays, on the display unit 148, information indicating a distribution of cells in the direction intersecting the longitudinal direction of the bioreactor 12 based on the electrostatic capacitance measured by the measurement unit 152. The storage control unit 158 executes storage control. For example, the storage control unit 158 stores, in the storage unit 144, information indicating the distribution of cells in the direction intersecting the longitudinal direction of the bioreactor 12 based on the electrostatic capacitance measured by the measurement unit 152.

The storage unit 144 may include a volatile memory (not illustrated) and a non-volatile memory (not illustrated). Examples of the volatile memory include a random access memory (RAM). The volatile memory is used as a working memory of the processor and temporarily stores data and the like necessary for processing or calculation. Examples of the non-volatile memory include a read only memory (ROM) and a flash memory. The non-volatile memory is used as a memory for storage and stores programs, tables, maps, and the like. At least a part of the storage unit 144 may be in the processor, the integrated circuit, or the like as described above.

The storage unit 144 can store information indicating the distribution of cells in the direction intersecting the longitudinal direction of the bioreactor 12 according to the storage control executed by the storage control unit 158 of the arithmetic unit 142. Note that a part of the storage unit 144 may be provided outside the measurement device 16.

The power supply unit 146 includes a power supply circuit capable of applying a voltage (or current) between the pair of electrodes 136. The power supply circuit includes a plurality of switches. Each of the switches switches connection and disconnection between a power supply (not illustrated) and the electrodes 136 according to a switching signal output from the arithmetic unit 142 (measurement unit 152).

The display unit 148 includes a drive circuit and a display. The display unit 148 can display information indicating the distribution of cells in the direction intersecting the longitudinal direction of the bioreactor 12 according to the display control executed by the arithmetic unit 142 (display control unit 156). For example, the display unit 148 may display the cell concentration in a numerical value. Alternatively, the display unit 148 may display the cell concentration as a graph.

### [3-2 Arrangement of Plurality of Electrodes 136]

The arrangement of the plurality of electrodes 136 (electrode groups 114) will be described below. Note that in the following description, the longitudinal direction of the bioreactor 12 is also referred to as the D1 direction. Among radial directions of the bioreactor 12, two directions that are perpendicular to each other are referred to as the D2 direction and the D3 direction. The D2 direction and the D3 direction are perpendicular to the D1 direction.

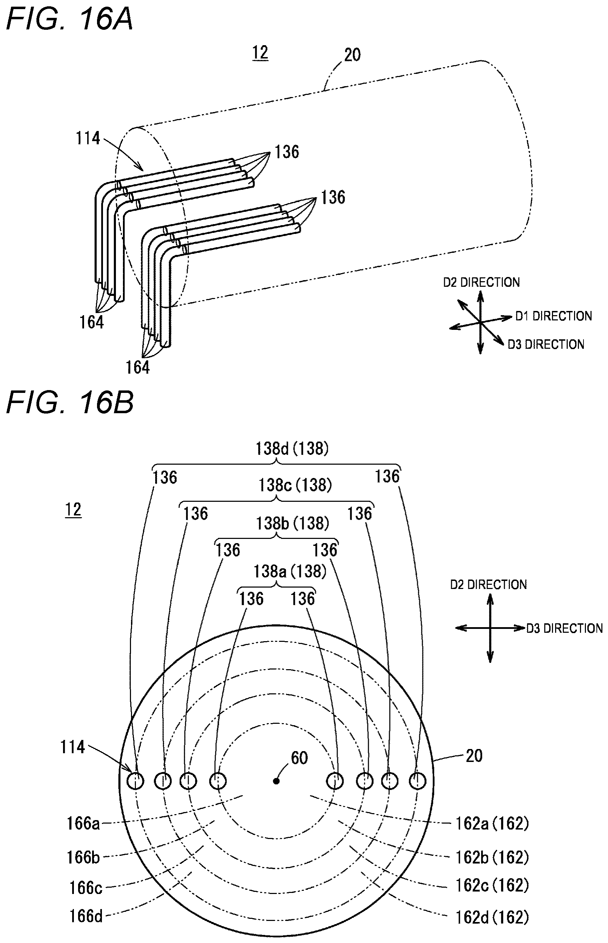

FIGS. 16A and 16B are schematic diagrams illustrating the arrangement of a plurality of electrodes 136 in the third embodiment. As illustrated in FIG. 16A, each of the electrodes 136 extends along the D1 direction. The electrodes 136 are disposed at the same position in the D1 direction. The electrode 136 is connected to the power supply unit 146 (FIG. 15) via a lead wire 164. As illustrated in FIG. 16B, a distance from each of two electrodes 136 of the plurality of electrodes 136 to the axis 60 of the housing 20 (bioreactor 12) is equal. The two electrodes 136 are spaced apart from each other to sandwich the axis 60 of the housing 20. That is, these two electrodes 136 and the axis 60 of the housing 20 are disposed along the radial direction (for example, D3 direction) of the housing 20 (bioreactor 12). When the electrostatic capacitance is measured, these two electrodes 136 are simultaneously selected. One of the two electrodes 136 is a positive electrode, and the other is a negative electrode. That is, when the electrostatic capacitance is measured, these two electrodes 136 form a pair. In this way, two electrodes 136 that form a pair in which only one electrode 136 is correlated with one electrode 136 in advance are referred to as an "assembled electrode 138". A plurality of assembled electrodes 138 having different distances between the electrodes 136 (inter-electrode distances) are provided inside the bioreactor 12.

As illustrated in FIGS. 16A and 16B, the plurality of assembled electrodes 138 (the plurality of electrodes 136) are disposed along the same direction (D3 direction). That is, the plurality of electrodes 136 are disposed along the direction (D3 direction) perpendicular to the longitudinal direction (D1 direction) of the bioreactor 12. Note that the plurality of electrodes 136 may be disposed along a direction intersecting the longitudinal direction (D1 direction) of the bioreactor 12 other than the D3 direction.

When the power supply unit 146 (FIG. 15) applies a voltage (or a current) to the assembled electrode 138 (the pair of electrodes 136), the assembled electrode 138 stores a charge corresponding to a cell concentration between the electrodes 136. Note that the cell concentration is substantially constant in a certain range centered on the assembled electrode 138. From the above, the assembled electrode 138 functions as a sensor member for measuring the cell concentration in a certain range. This certain range is referred to as a measurement range 162. As illustrated in FIG. 16B, the measurement range 162 has a substantially circular shape centered on the axis 60 of the housing 20 in the side view.

In the embodiment illustrated in FIGS. 16A and 16B, four assembled electrodes 138 are provided inside the bioreactor 12. Note that the number of assembled electrodes 138 may be two or more. Each of the electrodes 136 is disposed on any one of four concentric circles centered on the axis 60 of the housing 20. The electrode 136 in a first assembled electrode 138a is disposed on a circle having the smallest diameter. That is, an inter-electrode distance of the first assembled electrode 138a is the smallest among inter-electrode distances of the four assembled electrodes 138. The electrode 136 in a second assembled electrode 138b is disposed on a circle having the second smallest diameter. That is, an inter-electrode distance of the second assembled electrode 138b is the second smallest among the inter-electrode distances of the four assembled electrodes 138. The electrode 136 in a third assembled electrode 138c is disposed on a circle having the third smallest diameter. That is, an inter-electrode distance of the third assembled electrode 138c is the third smallest among the inter-electrode distances of the four assembled electrodes 138. The electrode 136 in a fourth assembled electrode 138d is disposed on a circle having the largest diameter. That is, an inter-electrode distance of the fourth assembled electrode 138d is the largest among the inter-electrode distances of the four assembled electrodes 138. The electrode 136 in the fourth assembled electrode 138d may be in contact with the inner peripheral surface of the housing 20.

Inside the bioreactor 12, four measurement ranges 162 are formed by the four assembled electrodes 138. The measurement range 162 (first measurement range 162a) of the first assembled electrode 138a is the smallest. The measurement range 162 (second measurement range 162b) of the second assembled electrode 138b is one size larger than the first measurement range 162a. The measurement range 162 (third measurement range 162c) of the third assembled electrode 138c is one size larger than the second measurement range 162b. The measurement range 162 (fourth measurement range 162d) of the fourth assembled electrode 138d is one size larger than the third measurement range 162c.

Here, the first measurement range 162a is also referred to as a first distribution range 166a. A range of the second measurement range 162b other than the first measurement range 162a is referred to as a second distribution range 166b. A range of the third measurement range 162c other than the second measurement range 162b is referred to as a third distribution range 166c. A range of the fourth measurement range 162d other than the third measurement range 162c is referred to as a fourth distribution range 166d. The cell concentration in these four distribution regions indicates a distribution of cells in a cross section perpendicular to the longitudinal direction of the bioreactor 12.

The number of cells in the measurement range 162 can be acquired based on the electrostatic capacitance in the measurement range 162. The number of cells in the first distribution range 166a can be acquired based on electrostatic capacitance in the first measurement range 162a. The number of cells in the second distribution range 166b can be acquired by subtracting the number of cells in the first measurement range 162a from the number of cells in the second measurement range 162b. The number of cells in the third distribution range 166c can be acquired by subtracting the number of cells in the second measurement range 162b from the number of cells in the third measurement range 162c. The number of cells in the fourth distribution range 166d can be acquired by subtracting the number of cells in the third measurement range 162c from the number of cells in the fourth measurement range 162d.

### [3-3 Operation of Distribution Measurement Device 10]

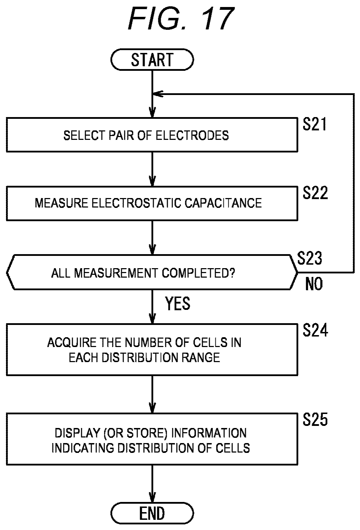

FIG. 17 is a flowchart illustrating the operation of the distribution measurement device 10 according to the third embodiment.

In step S21, the selection unit 150 selects a pair of electrodes 136 from the plurality of electrodes 136. For example, as illustrated in FIG. 16B and the like, when the assembled electrode 138 is formed in advance, the selection unit 150 selects the assembled electrode 138 for which measurement of the electrostatic capacitance is not completed.

In step S22, the measurement unit 152 executes switching control of the power supply unit 146 so that power is supplied to the pair of electrodes 136 selected by the selection unit 150. The power supply unit 146 performs switching according to a switching signal output from the measurement unit 152, and applies a voltage between the pair of electrodes 136. The measurement unit 152 measures electrostatic capacitance between the selected pair of electrodes 136.

In step S23, the determination unit 154 determines whether the measurement of all the electrostatic capacitance is completed. If the measurement of all the electrostatic capacitance is completed (step S23: YES), the processing proceeds to step S24. Meanwhile, if the measurement of some of the electrostatic capacitance is not completed (step S23: NO), the processing returns to step S21.

When the processing proceeds from step S23 to step S24, the measurement unit 152 converts the electrostatic capacitance into a cell concentration using a calibration curve that indicates a relationship between the electrostatic capacitance and the cell concentration. The storage unit 144 stores the calibration curve in advance. In a case of the arrangement of the electrodes 136 illustrated in FIGS. 16A and 16B, the measurement unit 152 acquires the number of cells in each of the first distribution range 166a to the fourth distribution range 166d based on the cell concentration in each of the first measurement range 162a to the fourth measurement range 162d.

In step S25, the display control unit 156 executes display control for displaying information indicating a distribution of cells in a direction intersecting the longitudinal direction (D1 direction) of the bioreactor 12. The display unit 148 displays information indicating the distribution of cells according to the display control. For example, the display unit 148 may display a graph or a numerical value as the information indicating the distribution of cells. Meanwhile, the storage control unit 158 stores, in the storage unit 144, information indicating the distribution of cells in the direction intersecting the longitudinal direction (D1 direction) of the bioreactor 12.

As described above, in the third embodiment, the longitudinal direction of the electrode 136 for measuring the electrostatic capacitance is along the longitudinal direction of the bioreactor 12. According to the third embodiment, by sequentially switching a combination of the electrodes 136, the distribution of cells in the direction intersecting the longitudinal direction of the bioreactor 12 can be measured. The distribution of cells in the direction intersecting the longitudinal direction of the bioreactor 12 contributes to optimization of cell culture.

### [4 Fourth Embodiment]

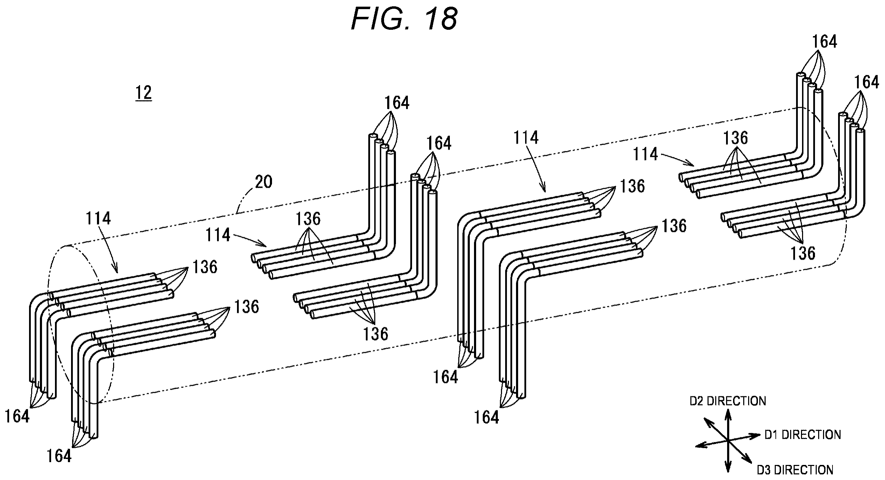

FIG. 18 is a schematic diagram illustrating an arrangement of a plurality of electrodes 136 in the fourth embodiment. As illustrated in FIG. 18, a plurality of electrodes 136 (a plurality of assembled electrodes 138) may be disposed at a plurality of positions in the longitudinal direction (D1 direction) of the bioreactor 12.

According to the fourth embodiment, a distribution of cells in the longitudinal direction (D1 direction) of the bioreactor 12 can be further measured.

### [5 Fifth Embodiment]

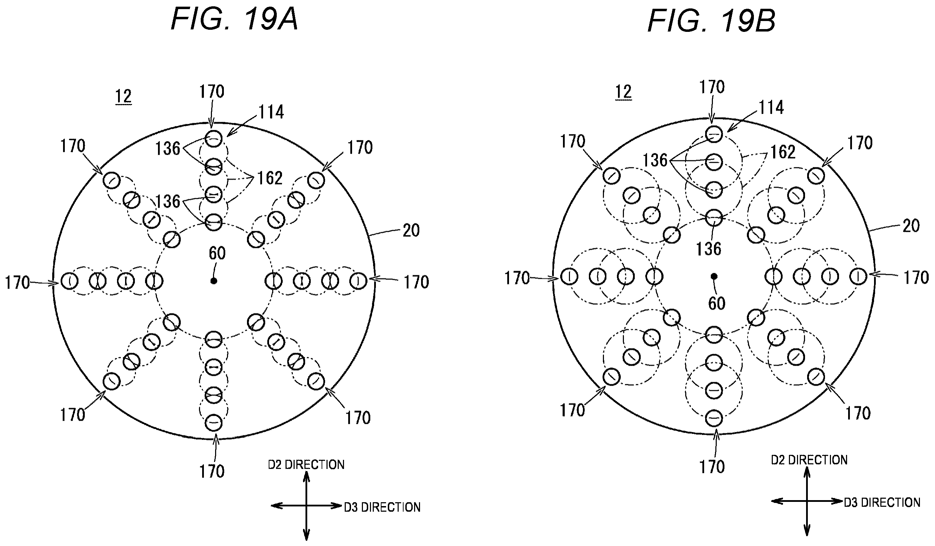

FIGS. 19A and 19B are schematic diagrams illustrating an arrangement of a plurality of electrodes 136 in the fifth embodiment. In the fifth embodiment, the electrode group 114 includes eight small electrode groups 170. Each of the eight small electrode groups 170 includes a plurality of electrodes 136. In FIGS. 19A and 19B, the small electrode group 170 includes four electrodes 136. A longitudinal direction of the electrode 136 is along the longitudinal direction of the bioreactor 12. That is, the electrode 136 extends along the longitudinal direction of the bioreactor 12. The electrode 136 is connected to the power supply unit 146 (FIG. 15). The electrodes 136 are disposed at the same position in the D1 direction.

In the small electrode group 170, the plurality of electrodes 136 and the axis 60 of the housing 20 are disposed along the radial direction of the housing 20 (bioreactor 12). A disposition direction of the small electrode group 170 is shifted by 45 degrees from a disposition direction of the adjacent small electrode groups 170 about the axis 60 of the housing 20.

In the fifth embodiment, the selection unit 150 (FIG. 15) can randomly select two electrodes 136 to form a pair of electrodes 136. For example, as illustrated in FIG. 19A, the selection unit 150 may sequentially select two electrodes 136 adjacent to each other from one small electrode group 170 as a pair of electrodes 136. As illustrated in FIG. 19B, the selection unit 150 may sequentially select two electrodes 136 sandwiching one or a plurality of electrodes 136 from one small electrode group 170 as a pair of electrodes 136. As illustrated in FIG. 19B, the selection unit 150 may sequentially select a pair of electrodes 136 from one small electrode group 170 such that a plurality of measurement ranges 162 overlap each other. As in the third embodiment, the selection unit 150 may select two electrodes 136 that are spaced apart from each other and sandwich the axis 60 therebetween.

According to the fifth embodiment, the pair of electrodes 136 can be formed in various combinations. Therefore, according to the fifth embodiment, the distribution of cells in the direction intersecting the longitudinal direction of the bioreactor 12 can be measured in more detail than in the third embodiment.

Note that, the invention is not limited to the above-described disclosure, and various configurations can be adopted without departing from the gist of the invention.

## Claims

1. A distribution measurement device comprising:
a plurality of electrodes provided in a bioreactor;
a selection unit that sequentially selects a pair of the electrodes from the plurality of electrodes; and
a measurement unit that sequentially measures a physical quantity between the pair of electrodes selected by the selection unit.

2. The distribution measurement device according to claim 1, wherein
the plurality of electrodes are disposed along a longitudinal direction of the bioreactor.

3. The distribution measurement device according to claim 2, wherein
an electrode group including the plurality of electrodes includes a plurality of assembled electrodes, and
the plurality of assembled electrodes are disposed apart from each other along the longitudinal direction of the bioreactor.

4. The distribution measurement device according to claim 2, wherein
a longitudinal direction of each of the electrodes intersects the longitudinal direction of the bioreactor.

5. The distribution measurement device according to claim 2, wherein
the physical quantity is electrostatic capacitance.

6. The distribution measurement device according to claim 2, wherein
a hollow fiber is provided in the bioreactor, and
a longitudinal direction of the hollow fiber is along the longitudinal direction of the bioreactor.

7. The distribution measurement device according to claim 2, wherein
the bioreactor is provided with a plurality of supply and discharge ports,
a first supply and discharge port of the plurality of supply and discharge ports is provided at one end of the bioreactor, and
a second supply and discharge port of the plurality of supply and discharge ports is provided at the other end of the bioreactor.

8. The distribution measurement device according to claim 2, further comprising:
a display control unit that displays, on a display unit, information indicating a distribution of cells in the longitudinal direction of the bioreactor based on the physical quantity measured by the measurement unit.

9. The distribution measurement device according to claim 2, further comprising:
a storage control unit that stores, in a storage unit, information indicating a distribution of cells in the longitudinal direction of the bioreactor based on the physical quantity measured by the measurement unit.

10. The distribution measurement device according to claim 2, further comprising:
a control unit that controls at least one of an inflow rate of a culture medium into the bioreactor and an outflow rate of the culture medium from the bioreactor such that a distribution of cells in the longitudinal direction of the bioreactor becomes uniform.

11. The distribution measurement device according to claim 1, wherein
a longitudinal direction of the electrode is along a longitudinal direction of the bioreactor.

12. The distribution measurement device according to claim 11, wherein
the physical quantity is electrostatic capacitance.

13. The distribution measurement device according to claim 11, wherein
a hollow fiber is provided in the bioreactor, and
a longitudinal direction of the hollow fiber is along the longitudinal direction of the bioreactor.

14. The distribution measurement device according to claim 11, further comprising:
a display control unit that displays, on a display unit, information indicating a distribution of cells in a direction intersecting the longitudinal direction of the bioreactor based on the physical quantity sequentially measured by the measurement unit.

15. The distribution measurement device according to claim 11, further comprising:
a storage control unit that stores, in a storage unit, information indicating a distribution of cells in a direction intersecting the longitudinal direction of the bioreactor based on the physical quantity sequentially measured by the measurement unit.
